# EUROPÄISCHE PATENTANMELDUNG

(11) **EP 0 779 110 A2**
(43) Veröffentlichungstag der Anmeldung: **18.06.1997**
(21) Anmeldenummer: 96118966.9
(22) Anmeldetag: 27.11.1996
(51) Int. Cl.: B08B 9/08

(54) **Verfahren zur Reinigung von Gebinden**

(30) Priorität: 11.12.1995 DE 19545967
(71) Anmelder: GEA Till GmbH & Co., D-65830 Kriftel (DE)
(72) Erfinder: Till, Volker, 65719 Hofheim am Taunus (DE)
(74) Vertreter: KEIL & SCHAAFHAUSEN Patentanwälte

(57) **Zusammenfassung**

Es wird ein Verfahren zur Reinigung von Gebinden, wie Kegs, beschrieben, das im wesentlichen die Arbeitsschritte des Resteentleerens, des Vorspülens, der Chemikalienreinigung, des Nachspritzens und des anschließenden Ausblasens und Sterilisierens mit Dampf umfaßt. Eine ausreichende und ausgesprochen kostengünstige Reinigung wird erfindungsgemäß dadurch ermöglicht, daß einzelne, vorzugsweise sämtliche Reinigungsschritte des Vorspülens, der Chemikalienreinigung und des Nachspritzens mit kalten Reinigungsmitteln durchgeführt werden.

## Beschreibung

Die Erfindung betrifft ein verfahren zur Reinigung von Gebinden, wie Kegs, mit folgenden Arbeitsschritten:
a) Resteentleerung,
b) Vorspülung, insbesondere mit Wasser,
c) ggf. mehrstufige Hauptreinigung mit Chemikalien, wie Laugen und/oder Säuren,
d) ggf. mehrstufiges Nachspritzen und
e) Ausblasen und Sterilisation mit Dampf.

In der Getränkeindustrie werden üblicherweise Fässer mit eingeschraubten, automatisch selbstschließenden Ventilen verwendet, für die sich die Bezeichnung Kegs eingebürgert hat. Derartige Behälter finden aber auch in der chemischen, der pharmazeutischen und allgemeinen Lebensmittelindustrie als Mehrweggebinde Verwendung. Sie werden im allgemeinen aus Aluminium oder Edelstahl hergestellt. Auch die Verwendung von Kunststoff ist möglich. Da in den obengenannten Industriezweigen außerordentlich hohe Ansprüche an die Sauberkeit der Gebinde gestellt werden, werden diese in einem mehrstufigen Reinigungszyklus gereinigt und anschließend sterilisiert. Hierzu werden im allgemeinen zunächst eventuelle Produktreste der vorhergehenden Füllung entleert und die Gebinde anschließend mit Wasser vorgespült, wobei meistens Heißwasser der Nachspritzung wiederverwendet wird. Dann erfolgt die Hauptreinigung durch Umwälzen von heißen Laugen und/oder Säuren, wobei üblicherweise zwei verschiedene Umwälzchemikalien nacheinander zum Einsatz kommen. Die Reinigungschemikalien werden daraufhin in einem ggf. mehrstufigen Nachspritzvorgang mit heißem Wasser ausgespült, bevor die Kegs mit Dampf ausgeblasen und sterilisiert werden. Nach der Reinigung und Sterilisation werden die Kegs abgekühlt, vorgespannt und befüllt. Um eine ausreichende Reinigung zu gewährleisten, werden die flüssigen Reinigungsmittel traditionsgemäß nur als Heißflüssigkeiten mit Temperaturen von wenigstens 85°C verwendet. Zum Aufheizen der Reinigungsflüssigkeiten ist jedoch ein erheblicher Energieaufwand notwendig. Außerdem wird aufgrund der großen Oberfläche der Reinigungsmittelbehälter, der Rohrleitungen und der zu reinigenden Fässer eine sehr große Wärmemenge an die Umgebung abgegeben. Hierbei ist zu berücksichtigen, daß der Reinigungszyklus für jedes Keg üblicherweise zirka fünf Minuten in Anspruch nimmt. Bei größeren Anlagen werden daher Kühltürme installiert, um die Wärmemenge abzuführen. Die Verwendung derart heißer Flüssigkeiten birgt außerdem naturgemäß erhebliche Verletzungsrisiken für das Bedienungspersonal der Anlage. Diese Nachteile werden jedoch in Kauf genommen, da davon ausgegangen wird, daß lediglich durch die hohen Temperaturen der Reinigungflüssigkeiten gewährleistet werden kann, daß eventuell in dem Gebinde verbliebene Produktreste vollständig entfernt werden.

Aufgabe der Erfindung ist es daher, die Reinigung von Gebinden kostengünstiger zu gestalten, wobei dennoch eine ausreichende Reinigung gewährleistet bleibt.

Diese Aufgabe wird mit der Erfindung im wesentlichn dadurch gelöst, daß wenigstens Teilschritte der Chemikalienreinigung und/oder des Nachspritzens mit kalten Reinigungsmitteln durchgeführt werden. Es hat sich überraschenderweise herausgestellt, daß die in dem Gebinde enthaltenen Produktreste auch mit kalten Reinigungsmitteln zuverlässig entfernt werden können. Die Keimfreiheit des Gebindes kann im Anschluß an den Reinigungsprozeß durch die Dampfsterilisation gewährleistet werden. Neben der Energieeinsparung durch den Verzicht auf das Erhitzen der Reinigungsmittel wird durch die kalten Reinigungsmittel auch ein Aufheizen der Metallmasse der Tanks und Rohrleitungen vermieden und die Wärmebelastung des Abwassers für die Gesamtbilanz eines Getränkebetriebes oder dgl. erheblich gesenkt. Bei größeren Anlagen sind die bisher notwendigen Kühltürme nicht mehr erforderlich. Somit werden die Investitions- und Betriebskosten der Reinigungsanlage erheblich reduziert. Außerdem werden die Verletzungsrisiken durch heiße Flüssigkeiten innerhalb der Anlage ausgeschlossen.

Sollen bspw. zur Verbesserung der chemischen Reaktionen während der Hauptreinigung einzelne Teilschritte der Reinigung nicht mit kalten Reinigungsmitteln durchgeführt werden, so erfolgt hierbei erfindungsgemäß nur eine moderate Erwärmung der Reinigungsmittel, bspw. auf 30 bis 50 °C.

Die Hauptvorteile der Erfindung werden jedoch dann erzielt, wenn die gesamte Hauptreinigung und das Nachspritzen und vorzugsweise auch die Vorspülung mit kalten Reinigungsmitteln durchgeführt wird. Wie sich in Versuchen ergeben hat, läßt sich auch mit durchweg kalten Reinigungsmitteln eine ausreichende Reinigung erreichen, wobei ein Maximum an Energie eingespart wird.

Zum Erreichen einer ausreichenden Sterilität der Metalloberfläche des zu reinigenden Gebindes ist es erforderlich, die sich an die Reinigung anschließende Sterilisation in Abhängigkeit von der Sterilisationstemperatur über eine bestimmte Dauer durchzuführen. So muß bspw. bei einer Sterilisationstemperatur von 63°C die Metalloberfläche ca. 15 Minuten dieser Temperatur ausgesetzt werden, während dies bei einer Sterilisationstemperatur von 72°C lediglich für etwa 30 Sekunden notwendig ist. Üblicherweise wird das Verhältnis von Sterilisationstemperatur und -zeit in Pasteur-Einheiten gemessen. Gemäß einer bevorzugten Ausgestaltung der Erfindung ist daher vorgesehen, daß sämtliche für eine ausreichende Sterilisation des Gebindes notwendigen Pasteur-Einheiten während der für das Ausblasen der Nachspritzflüssigkeit und die anschließende Sterilisation vorgesehenen Dämpfzeit aufgebracht werden.

Weiterbildungen, Vorteile und Anwendungsmöglichkeiten der Erfindung ergeben sich auch aus der nachfolgenden Beschreibung eines Ausführungsbeispiels. Dabei bilden alle beschriebenen Merkmale für sich oder in beliebiger Kombination den Gegenstand der Erfindung, unabhängig von ihrer Zusammenfassung in den Ansprüchen oder deren Rückbeziehung.

Die Reinigung von Mehrweggebinden, wie Kegs, erfolgt erfindungsgemäß unter Anwendung der bisher üblichen Schritte des Resteentleerens, Vorspritzens mit einer Vorspritzflüssigkeit, bspw. dem in der Nachspritzphase ablaufenden Wasser, einer Umwälzreinigung mit Chemikalien, eines Nachspritzens mit Frischwasser und einer anschließenden Dampfsterilisation. Die Chemikalienreinigung kann in mehreren Schritten, üblicherweise mit zwei verschiedenen Umwälzchemikalien durchgeführt werden. Auch das Nachspritzen kann in mehreren aufeinanderfolgenden Phasen erfolgen.

Sämtliche Reinigungsschritte des Vorspritzens, der Chemikalienreinigung und des Nachspritzens werden mit kalten Reinigungsmitteln durchgeführt, so daß ein Aufheizen der Behälter und der Rohrleitungen der Anlage vermieden wird.

Selbstverständlich ist es auch möglich, einzelne Stufen des Reinigungsprozesses, bspw. die Säurereinigung, mit erwärmten Reinigungsmitteln durchzuführen, wobei im allgemeinen eine moderate Erwärmung auf ca. 40 °C ausreicht. Dennoch kann die Mehrzahl oder jedenfalls einzelne Reinigungsschritte mit kalten Reinigungsmitteln durchgeführt werden.

Das Ausblasen der Nachspritzflüssigkeit und die Sterilisation mit Dampf erfolgen über eine ausreichende Dauer, so daß die für eine ausreichende Sterilisation des Gebindes notwendigen Pasteur-Einheiten vollständig während der Dämpfzeit erreicht werden.

Mit der Erfindung wird somit eine vollständige Reinigung und Sterilisation von Mehrweggebinden, wie Kegs, ermöglicht, wobei gegenüber herkömmlichen Verfahren die zum Aufheizen der Reinigungsmittel und Abführen der überschüssigen Wärme notwendigen Energien und Installationen eingespart werden können. Hierdurch werden die Kosten für die Errichtung und das Betreiben entsprechender Reinigungsanlagen erheblich gesenkt.

## Patentansprüche

1. Verfahren zur Reinigung von Gebinden, wie Kegs, mit folgenden Arbeitsschritten:
a) Resteentleerung,
b) Vorspülung, insbesondere mit Wasser,
c) ggf. mehrstufige Hauptreinigung mit Chemikalien, wie Laugen und/oder Säuren,
d) ggf. mehrstufiges Nachspritzen, und
e) Ausblasen und Sterilisation mit Dampf,
**dadurch gekennzeichnet**, daß wenigstens Teilschritte der Hauptreinigung und/oder des Nachspritzens mit kalten Reinigungsmitteln durchgeführt wird.

2. Verfahren nach Anspruch 1, **dadurch gekennzeichnet**, daß bei nicht mit kalten Reinigungsmitteln durchgeführten Teilschritten der Hauptreinigung und/oder des Nachspritzens nur eine moderate Erwärmung der Reinigungsmittel, bspw. auf 40 °C, erfolgt.

3. Verfahren nach Anspruch 1, **dadurch gekennzeichnet**, daß die Hauptreinigung und/oder das Nachspritzen ausschließlich mit kalten Reinigungsmitteln durchgeführt wird.

4. Verfahren nach einem der Ansprüche 1 bis 3, **dadurch gekennzeichnet,** daß die Vorspülung mit kalten Reinigungsmitteln durchgeführt wird.

5. Verfahren nach einem der Ansprüche 1 bis 4, **dadurch gekennzeichnet**, daß sämtliche Reinigungsschritte der Vorspülung, Hauptreinigung und des Nachspritzens mit kalten Reinigungsmitteln durchgeführt werden.

6. Verfahren nach einem der Ansprüche 1 bis 5, **dadurch gekennzeichnet**, daß sich sämtliche für eine ausreichende Sterilisation des Gebindes notwendigen Pasteur-Einheiten während der Dämpfzeit in Arbeitsschritt e) ergeben.
